# EUROPÄISCHE PATENTSCHRIFT

(11) **EP 0 494 440 B1**
(45) Veröffentlichungstag und Bekanntmachung des Hinweises auf die Patenterteilung: **09.08.1995**
(21) Anmeldenummer: 91122148.9
(22) Anmeldetag: 23.12.1991
(51) Int. Cl.: C07C 51/14

(54) **Kontinuierliches Verfahren zur Herstellung von Isobuttersäure durch Koch'sche Synthese**
Continuous process for preparing isobutyric acid by Koch's synthesis
Procédé continu pour la préparation d'acide isobutyrique par la synthèse de Koch

(30) Priorität: 09.01.1991 DE 4100420
(43) Veröffentlichungstag der Anmeldung: 15.07.1992
(73) Patentinhaber: Röhm GmbH, 64293 Darmstadt (DE)
(72) Erfinder: Ruppert, Wolfgang, Dr.-Ing., W-6101 Bickenbach (DE); Plösser, Willi, W-6104 Seeheim-Jugenheim 3 (DE)

(56) Entgegenhaltungen:
- EP-A- 0 310 878
- US-A- 2 831 877
- US-A- 3 661 951

## Beschreibung

Die Erfindung betrifft ein kontinuierliches Verfahren zur Herstellung von Isobuttersäure durch Koch'sche Synthese aus Propen, Kohlenmonoxid und Wasser in Fluorwasserstoff als Koch'schem Katalysator unter Druck.

### Stand der Technik

Die Koch'sche Synthese von organischen Carbonsäuren aus Olefinen, Kohlenmonoxid und Wasser in einem stark sauren Reaktionsmedium ist in der US-Patentschrift 2 831 877 in ihren Grundzügen beschrieben; ein Beispiel ist die Umsetzung von Propen zu Isobuttersäure. Der Einsatz von wasserfreiem Fluorwasserstoff als Reaktionsmedium wird an einem anderen Olefin beispielhaft beschrieben. Die Umsetzung wurde bei einer Temperatur von 20 bis 30°C unter einem Druck von 25 bis 35 atm durchgeführt. Das Gewicht des für die Reaktion erforderlichen Autoklaven übersteigt das des Reaktionsgemisches um das Vielfache. Dadurch wird die entstehende Reaktionswärme von dem Reaktionsgefäß rasch aufgenommen, so daß es nicht zu dem Temperaturanstieg kommt, der bei einer adiabatischen Umsetzung auftreten würde.

Y. Takezaki (Bulletin of the Japan Petroleum Institute, Vol.8, 1966, 31-38) hat die Abhängigkeit der Ausbeute an Isobuttersäure bei der Koch'schen Synthese aus Propen, Kohlenmonoxid und Wasser in Fluorwasserstoff als Koch'schem Katalysator unter Druck in Abhängigkeit von den Verfahrensparametern untersucht und ein Optimum beim Einsatz von 15 Mol Fluorwasserstoff je Mol Propen, einem Wassergehalt von 20 Gew.-%, einer Temperatur von 94°C und einem Druck von 190 bar ermittelt.

Bei der kontinuierlichen Durchführung dieser Reaktion erhält man gemäß der DE-C 30 33 655 eine hohe Ausbeute und Selektivität an Isobuttersäure, wenn das reagierende Gemisch unter hoher Rückvermischung bei einer Temperatur zwischen 80 und 160°C umgesetzt wird und die Anteile von Propen und Wasser im Reaktionsgemisch auf einen niedrigen Wert begrenzt werden. Durch diese Verfahrensweise wird auch die Bildung von hochsiedenden Nebenprodukten unterdrückt.

Es hat sich jedoch gezeigt, daß auch bei dieser Verfahrensweise störende Nebenprodukte entstehen. In der gewonnenen Isobuttersäure finden sich höhere Ketone in einer Konzentration von 1 bis 2 Gew.-% und lassen sich nur durch umständliche mehrstufige Verfahren abtrennen. Bei der oxydativen Dehydrierung dieser Isobuttersäure zu Methacrylsäure bewirkt die Verunreinigung durch Ketone eine Umsatzminderung bis zu 15 %, was nicht tragbar ist.

### Aufgabe und Lösung

Der Erfindung liegt die Aufgabe zugrunde, bei der kontinuierlichen Herstellung von Isobuttersäure durch Koch'sche Synthese aus Propen, Kohlenmonoxid und Wasser in Fluorwasserstoff als Koch'schem Kataylator unter Druck die Bildung der höheren Ketone zu vermeiden oder zu vermindern. Überwiegend entstehen C₇-Ketone, die möglicherweise durch Fluorwasserstoffabspaltung zwischen intermediär gebildetem Isobuttersäurefluorid und Propen oder durch die Einwirkung des entsprechenden Acylkations auf Propen entstehen. Eine Möglichkeit zur systematischen Unterdrückung dieser Nebenreaktion war nicht zu erkennen.

Es wurde nun gefunden, daß die Bildung der Ketone stark unterdrückt wird, wenn man die Ausgangsstoffe bei einer Temperatur unter 30°C unter Druck mischt, das Reaktionsgemisch ohne Rückvermischung umsetzt und seine Temperatur auf wenigstens 60°C ansteigen läßt. Die bei der Umsetzung entstehende Reaktionswärme reicht aus, um diesen Temperaturanstieg hervorzurufen, wenn die Umsetzung im wesentlichen adiabatisch durchgeführt wird.

Der überraschende Auswirkung des nicht-isothermen Reaktionsverlaufs ist möglicherweise so zu deuten, daß die unerwünschten höheren Ketone überwiegend in einer kritischen Anfangsphase der Umsetzung bei höherer Temperatur gebildet werden. Bei einer niedrigen Anfangstemperatur wird diese Nebenreaktion offenbar unterdrückt. Nach Überwindung dieser Anfangsphase wird die weitere Umsetzung durch die Selbsterwärmung des Gemisches stark beschleunigt, ohne daß dabei die zu Ketonen führenden Nebenreaktionen wieder aufleben.

### Bevorzugte Ausführung der Erfindung

Propen, Kohlenmonoxid, Wasser und Fluorwasserstoff werden vorzugsweise in Molverhältnissen von 1 : 1-20 : 0,7-1,0 : 8-30 kontinuierlich in den Reaktionsraum eingeführt. Dies kann über getrennte, in den Eingangsraum des Reaktor mündende Rohrleitungen geschehen. Man kann jedoch auch die flüssigen Ausgangsstoffe vorher mischen und die Mischung mit einer Pumpe in den Reaktor einführen. Im Reaktor stellt sich ein Gesamtdruck von etwa 10 bis 250 bar ein. Die Wassermenge wird an Hand der Zusammensetzung des ausgetragenen Reaktionsgemisches so hoch bemessen, daß sie gerade verbraucht wird und der entstehenden Isobuttersäure stets äquivalent bleibt. Ein Verminderung der Wassermenge würde eine Ausbeuteverminderung nach sich ziehen. Eine höhere Wassermenge würde zu einem Anstieg des Wassergehalts im Reaktionsgemisch führen und dessen korrosive Eigenschaften verstärken.

Die Umsetzung ohne Rückvermischung setzt voraus, daß neu eingeführte kalte Ausgangsstoffe nicht mit dem schon reagierenden oder bereits ausreagierten heißen Reaktorinhalt vermischt werden. Diese Voraussetzung läßt sich in der Praxis nur näherungsweise erfüllen. Das Ziel der Erfindung wird erreicht, wenn man die Ausgangsstoffe kontinuierlich in einen Rohrreaktor einleitet und für eine sogenannte "Pfropfenströmung" sorgt. Reaktoreinbauten, die zu einer axialen Verwirbelung des Reaktionsgemisches oder zur Aufteilung in Teilströme und deren Wiedervereinigung vor Abschluß der Umsetzung führen, sind in der Regel nachteilig. Vorteilhaft ist die Verwendung eines aufrecht stehenden, schlanken Rohrraktors mit glatten Innenwänden, der von unten nach oben durchströmt wird. Seine Schlankheit, d.h. das Verhältnis von Höhe zu lichter Weite, beträgt vorzugsweise 5 : 1 bis 300 : 1. Der Durchmesser des Rohrreaktors liegt vorteilhaft bei 1 bis 5 cm. Bei größerem Gesamtquerschnitt ist es vorteilhaft, den Reaktorraum durch senkrecht eingebaute Trennwände in vertikaler Richtung zu unterteilen, um einer axialen Vermischung entgegenzuwirken.

Eine hohe Ausbeute an Isobuttersäure wird erreicht, wenn die Anfangstemperatur der Ausgangsstoffe niedrig und die Endtemperatur des Reaktionsgemisches hoch ist. Anfangstemperaturen von 0 bis 20°C sind besonders vorteilhaft. Der Temperaturanstieg durch die freiwerdende Reaktionswärme hängt, sofern nicht durch Kühlung Wärme entzogen wird, vom Molverhältnis von Fluorwasserstoff zu Propen ab. Endtemperaturen von 70 bis 100°C sind vorteilhaft. Im gleichbleibenden Betriebszustand (steady state) steigt die Temperatur im Reaktor vom Eingang zum Ausgang stetig an. Der Reaktor steht im thermischen Gleichgewicht mit dem Reaktionsgemisch. Die Wärmeverluste durch die Reaktorwand sollten durch eine gute Isolierung beschränkt werden. Gegebenenfalls kann die Temperatur im oberen Teil des Reaktors durch zusätzliche Erwärmung weiter gesteigert und damit die Reaktion noch mehr beschleunigt werden.

Die Verweilzeit des Gemisches im Reaktor beträgt in der Regel 10 bis 600 s, vorzugsweise 60 bis 150 s. Aus dieser niedrigen Verweilzeit ergibt sich eine höhere Raum-Zeit-Ausbeute als bei anderen kontinuierlichen Verfahren zur Herstellung von Isobuttersäure; sie kann im Bereich von 2 bis 15 Liter Isobuttersäure je Liter Reaktorvolumen und Stunde liegen.

Am Ausgang des Reaktors wird das Reaktionsgemisch über ein Entspannungsventil kontinuierlich ausgetragen und in an sich bekannter Weise in seine Bestandteile zerlegt. Kohlenmonoxid und Fluorwasserstoff werden zurückgeführt. Isobuttersäure kann durch Destillation mit einem Gehalt an höheren Ketonen unter 0,5 Gew.-% gewonnen werden. Sie eignet sich ohne weitere Reinigung zur oxydativen Dehydrierung zu Methacrylsäure.

### BEISPIEL

In ein aufrecht stehendes, druckfestes Reaktionsrohr von 3 m Länge und einem lichten Durchmesser von 21 mm wurden kontinuierlich pro Stunde 20 kg Ausgangsstoffe eingepumpt. Die molare Zusammensetzung der Ausgangsstoffe war:
Propen : CO : H₂0 : HF = 1 : 3,8 : 0,92 : 20.
Die Eintrittstemperatur betrug 5^{o}C, die Austrittstemperatur 87°C, der Druck 140 bar. Die Verweilzeit lag bei 1 min; daraus ergibt sich eine Raum-Zeit-Ausbeute von 3,5 l IBA/l.h.
Am Kopf des Rohrreaktors wurde das Gemisch über ein Entspannungsventil kontinuierlich ausgetragen, wobei sich CO und HF größtenteils gasförmig abschieden. In einer Destillationskolonne wurden die niedrigsiedenden Bestandteile bei Normaldruck und einer Sumpftemperatur von 165°C vollständig abgetrennt. Der Sumpfablauf wurde über eine 40-bödige Kolonne bei 800 mbar destilliert. Gaschromatographisch wurden in der als Destillat erhaltenen Isobuttersäure 0,2 Gew.-% C₇-Ketone gefunden, darunter verschiedene ungesättigte Isomere C₇H₁₂O vom Mol.Gew. 112, wie 2-Methyl-hexen-4-on-3, Hepten-2-on-4, 2,4-Dimethylpenten-1-on-3 und 2-Methylhexen-1-on-3.

Zum Vergleich wurde das gleiche Gemisch von Ausgangsstoffen kontinuierlich in einen 1,7 l-Rührautoklaven eingepumpt. Die Reaktortemperatur wurde bei 115°C gehalten. Die Verweilzeit betrug hier 3,75 min. Das Reaktionsgemisch wurde wie oben entspannt und aufgearbeitet.
Die Ausbeute an Isobuttersäure betrug bei beiden Versuchen 92 %. Die beim Vergleichsversuch erhaltene Isobuttersäure enthielt jedoch 1,7 Gew.-% der erwähnten C₇-Ketone.

## Patentansprüche

1. Kontinuierliches Verfahren zur Herstellung von Isobuttersäure durch Koch'sche Synthese aus Propen, Kohlenmonoxid und Wasser in Fluorwasserstoff als Koch'schem Katalysator unter Druck,
dadurch gekennzeichnet,
daß man die Ausgangsstoffe bei einer Temperatur unter 30°C unter Druck mischt, das Reaktionsgemisch ohne Rückvermischung umsetzt und seine Temperatur auf wenigstens 60°C ansteigen läßt.

2. Verfahren nach Anspruch 1, dadurch gekennzeichnet, daß das Reaktionsgemisch nach einer Verweilzeit von 10 bis 600 Sekunden entspannt wird.

## Claims

1. A continuous process for preparing isobutyric acid by Koch's synthesis from propene, carbon monoxide and water in hydrogen fluoride as Koch's catalyst under pressure, characterised in that the starting substances are mixed under pressure at a temperature of below 30°C, the reaction mixture is reacted without back mixing and its temperature is allowed to increase to at least 60°C.

2. A process according to claim 1, characterised in that the pressure of the reaction mixture is released after a residence time of 10 to 600 seconds.

## Revendications

1. Procédé continu de préparation d'acide isobutyrique par la synthèse de Koch à partir de propène, de monoxyde de carbone et d'eau dans de l'acide fluorhydrique servant de catalyseur de Koch et sous pression,
caractérisé en ce que l'on mélange les substances de départ à une température inférieure à 30°C et sous pression, on fait réagir le mélange réactionnel sans remélangeage et on laisse sa température s'élever à 60°C au moins.

2. Procédé selon la revendication 1, caractérisé en ce que le mélange réactionnel est détendu après une durée de séjour de 10 à 600 s.
